## Europäisches Patentamt
## European Patent Office
## Office européen des brevets

(19)

(11) Publication number: **0 002 313**
**B1**

(12) **EUROPEAN PATENT SPECIFICATION**

(45) Date of publication of patent specification: **21.10.81**

(51) Int. Cl.³: **C 07 C 2/62, C 07 C 7/04**

(21) Application number: **78300523.4**

(22) Date of filing: **20.10.78**

(54) Process for catalytic alkylation; process for the separation of the alkylation reactor effluent.

(30) Priority: **26.10.77 US 845762**

(43) Date of publication of application:
**13.06.79 Bulletin 79/12**

(45) Publication of the grant of the European patent:
**21.10.81 Bulletin 81/42**

(84) Designated Contracting States:
**BE CH DE FR GB LU NL SE**

(56) References cited:
**FR - A - 2 338 236**
**US - A - 3 234 301**

(73) Proprietor: **THE STANDARD OIL COMPANY**
**Midland Building**
**Cleveland, Ohio 44115 (US)**

(72) Inventor: **Jones, Richard Howard**
**1432 Dorsh Road**
**South Euclid Ohio 44121 Cuyahoga Country (US)**

(74) Representative: **Stagg, Diana Christine**
**ELKINGTON & FIFE High Holborn House 52-54**
**High Holborn**
**London WC1V 6SH (GB)**

Courier Press, Leamington Spa, England.

Process for catalytic alkylation; process for the separation of the alkylation reactor effluent.

This invention relates to an improved process for the catalytic alkylation of isobutane with an olefin.

The catalytic alkylation of isobutane is well known in the art and involves the reaction of an olefin with isobutane in the presence of an acid catalyst to produce high octane branched chain hydrocarbons (alkylate) for use in aviation gasoline and motor fuel. Specifically, the olefin is combined with isobutane in the presence of an acid catalyst in a reactor and undergoes an exothermic reaction. The acid is then separated from the reactor effluent. After acid separation, the reactor effluent proceeds to a series of distillation columns to separate the inert alkanes, the unreacted isobutane for recycling, and to recover the alkylate. Some of the effluent is treated to remove residual acid and undesired reaction products. A variation of this process has been to use the vaporization of a portion of the reactor effluent to cool the reactor, and has been described in *Hydrocarbon Processing,* September, 1974, page 206.

The vapours produced by cooling the reactor have been used to supply heat to a deisobutanizer distillation column. U.S. Patent No. 3,162,694 and U.S. Reissue Patent No. 26,060 disclosed such a process. These processes however, suffer from several disadvantages.

First, the amount of vapours produced by cooling the reactor are not normally sufficient to supply the total heat necessary to the deisobutanizer. Secondly, the normally high operating pressures of the distillation columns make it difficult to use these vapours as a source of heat.

A process for the catalytic alkylation of isobutane with an olefin, in which the reactor effluent is used to provide cooling for the condensers used, is described in our French Patent No. 2,338,236.

The present invention reduces the disadvantages of the known processes by using the reactor effluent to cool the overhead vapour streams of the distillation columns used in the process. This allows the distillation columns to operate at a much lower pressure. The increased vapours of the reactor effluent produced by cooling the distillation column overheads, along with the lower operating pressure of these columns, allow the total energy input to the process to be greatly reduced.

The invention therefore provides a process for the catalytic alkylation of isobutane with an olefin in which:

(a) the olefine is contacted with a molar excess of isobutane and inert alkanes in the presence of an acid catalyst in a reactor to form a reactor effluent containing alkylate, isobutane, acid catalyst and inert alkanes;

(b) the acid catalyst is separated from the reactor effluent to form a hydrocarbon mixture;

(c) a liquid vapour separation is effected on the hydrocarbon mixture to obtain a liquid bottoms stream containing isobutane and alkylate and an overhead vapour stream containing isobutane and inert alkanes, and

(d) the liquid bottoms stream is heated in a distillation zone to form a liquid bottoms product containing alkylate and a vapour distillation effluent containing isobutane separated from the alkylate;

(e) a portion of the hydrocarbon mixture formed in step (b) is passed as a cooling effluent in indirect heat exchange with the vapour distillation effluent obtained in step (d) to condense isobutane and to vapourise at least a portion of the cooling effluent; and

(f) the cooling effluent is admixed with the hydrocarbon mixture formed in step (b) so that the cooling effluent is subjected to liquid vapour separation together with the hydrocarbon mixture in step (c) characterised in that the overhead vapour stream produced in step (c) is compressed in a compressor to obtain a compressed vapour stream and at least a portion of the compressed vapour stream is passed in indirect heat exchange relation with the liquid in the distillation zone of (d) to condensate at least a portion of the compressed vapour stream and thereby supply heat to the distillation zone.

The invention further provides a process for separating inert alkanes and isobutane from a gaseous alkylation reactor effluent containing alkylate, isobutane and inert alkanes in which:

(a) the liquid vapour separation is effected on a portion of the reactor effluent to obtain an overhead vapour stream containing isobutane and inert alkanes and a liquid bottoms stream containing isobutane and alkylate; and

(b) the overhead vapour stream is compressed; which process is characterised in that,

(c) at least a portion of the compressed overhead vapour stream is passed through a heat exchanger thereby at least partially condensing the compressed overhead vapour stream to form an exchanger effluent stream;

(d) a first portion of the exchanger effluent stream is passed to an alkane distillation zone in which the liquid is heated by passing it in indirect heat exchange with the heat exchanger of (c), thereby distilling and separating inert alkane as a vapour fraction and isobutane as liquid bottoms product;

(e) a portion of the reactor effluent is passed as a cooling effluent in indirect heat exchange with the vapour fraction of (d) to condense the inert alkane therein and to vapourise a portion of the reactor effluent; and

(f) the cooling effluent is mixed with the reactor effluent so that the cooling effluent is subjected to liquid vapour separation together with the reactor effluent in step (a).

Using the process of the present invention the amount of isobutane that must be treated and recovered in the distillation column is greatly reduced, cooling water is no longer necessary for the distillation column overhead condenser, the distillation columns may be operated at substantially lower pressures, and a much greater heat transfer rate may be achieved between the compressed vapour stream and the distillation columns. Further, the compressed vapour stream can now supply heat to columns other than the deisobutanizer.

The invention will now be further described with reference to the accompanying drawings.

Figure 1 shows the process as applied to a deisobutanizer distillation column.

Figure 2 shows the process as applied to a depropanizer distillation column.

Figure 3 shows an alternative embodiment of the process of Figure 2.

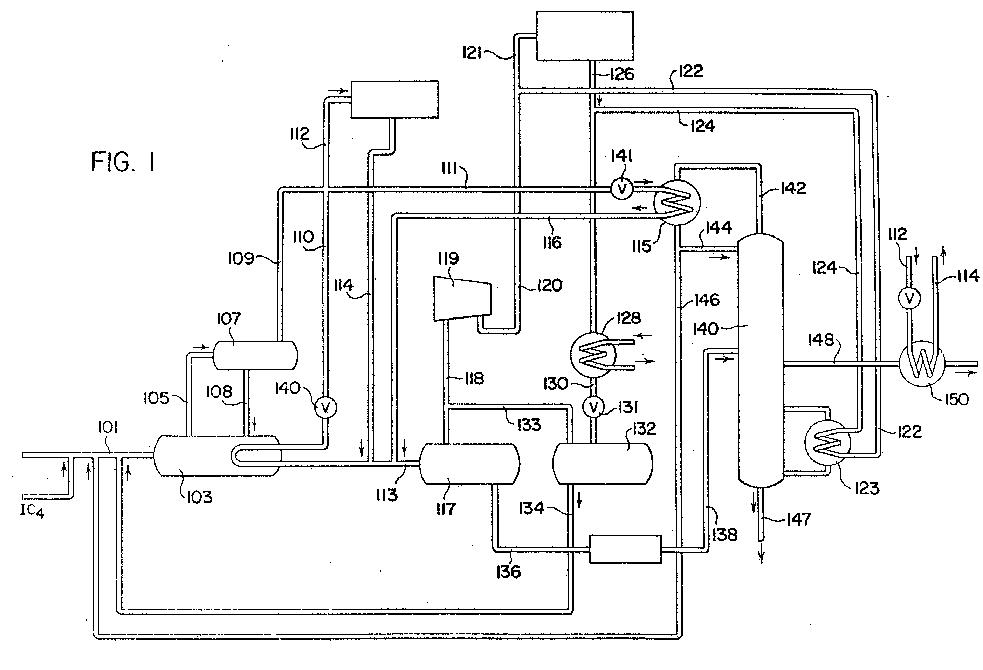

Referring to Figure 1, olefin and isobutane feed, in liquid phase, are combined in line 101 and fed to reactor 103 where the reactants are mixed with acid catalyst and reacted to form alkylate. The product leaves the reactor through line 105 to the acid separator 107. Here the acid is separated from the hydrocarbon mixture and returned to the reactor through line 108. The hydrocarbon mixture containing alkylate, isobutane and inert alkanes leaves the acid separator through line 109.

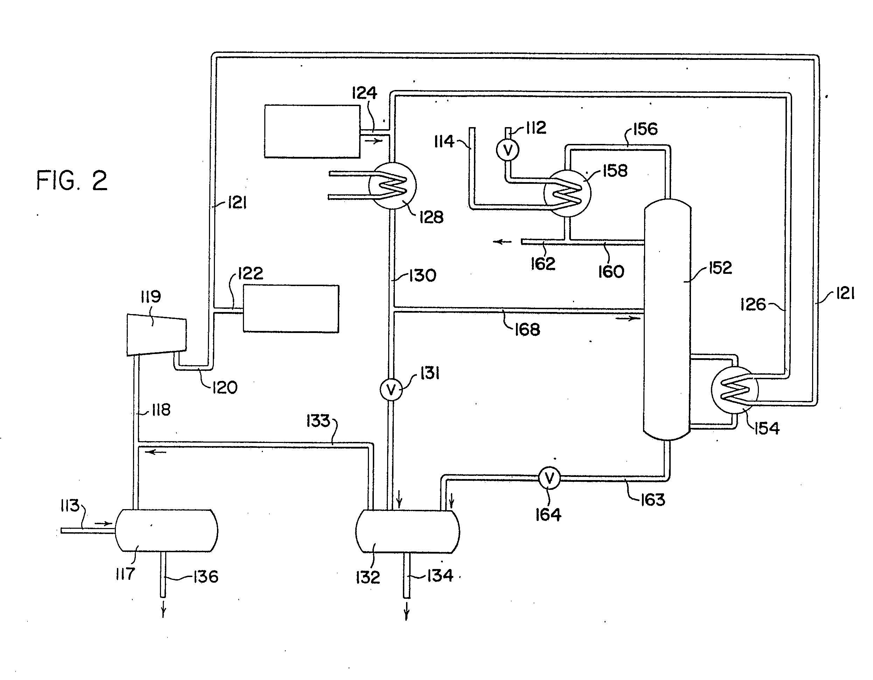

The hydrocarbon mixture goes to provide cooling in the condensers through line 111 and 112. Specifically referring to line 111, it is seen how the cooling function of the present invention is applied to the deisobutaniser column. Hydrocarbon mixture is transmitted through line 111 and reduced in pressure by valve 141. The mixture is then transmitted to condenser 115 where at least part of the hydrocarbon mixture is vapourised to provide cooling in the condenser. The hydrocarbon mixture, after providing cooling, is transmitted through line 116 to the vapour-liquid separator 117. Cooling may also be supplied to other condensers such as the depropaniser, shown in greater detail in Figure 2, or the later described butane sidestream drawn from the deisobutaniser column, by taking the hydrocarbon mixture through line 112, vapourising at least part of the hydrocarbon mixture in the condenser, and transmitting the condenser effluent through line 114 to the vapour liquid separator 117. The hydrocarbon mixture can also be used to cool the reactor through line 110, being let down on pressure through valve 140. It is then transmitted through line 113 to the vapour liquid separator.

In the vapour liquid separator 117 a vapour liquid separation takes place. The vapour contains primarily isobutane and inert alkanes, and the liquid contains primarily isobutane and alkylates. However, some n-butane may be present in the liquid because of the boiling point of n-butane. The vapours are transmitted through line 118 to a vapour compressor 119. The compressed vapours leave the compressor through line 120. The compressed vapours then go to provide heating in exchangers through lines 121 and 122. Specifically referring to line 122, it is seen how the heating function of the present invention is applied to the deisobutaniser column. The compressed vapours are transmitted through line 122 to a heat exchanger 123 wherein at least part of the vapours are condensed to provide heat in the exchanger. The compressed vapours, after providing heat, are transmitted through line 124 to a condenser 128. This condenser effects a condensation of the remaining vapours, and the condensed, compressed vapours are then transmitted through line 130 to a second vapour liquid separator 132. In the same manner, heat is supplied to the other exchangers, such as the depropaniser heat exchanger, by taking the compressed vapours through line 121, condensing at least part of the vapours in the exchanger and transmitting the exchanger effluent through line 126. This stream is then combined with line 124 prior to condenser 128.

The pressure in line 130 is maintained by valve 131. The condensed stream leaving condenser 128 through line 130 is reduced in pressure and thus partially vaporised by valve 131 prior to its entry into the second vapour liquid separator 132. Vapours are removed from the separator through line 133 and combined with line 118 back to the compressor. These vapours may be returned to the suction of the compressor, or some intermediate point within the compressor stages. The liquid from the second vapour liquid separator contains isobutane and some inert alkanes and is transmitted through line 134 back to the reactor to provide excess isobutane for the reaction.

The liquid from the first vapour liquid separator 117 containing isobutane and alkylate proceeds through line 136 to a treating section where residual acid and acidic compounds are removed. After treating, the liquid is transmitted through line 138 to the deisobutaniser column 140. Heat is applied to the column through heat exchanger 123 from the compressed vapours as described previously. Isobutane is distilled and exits the top of the column as a vapour in line 142. This vapour is condensed in the overhead condenser 115 and the condensed isobutane is then split for reflux to the column through line 144, and is recycled to the reactor through line 146. The alkylate product is recovered as a liquid bottoms stream from the column through line 147.

Also shown on the drawing is the optional use of recovering butane as a sidestream draw from the deisobutaniser column 140. Vapours are removed through line 148 and transmitted to condenser 150. The reactor effluent can be used as the cooling medium through line 112 and 114 to condense the butane. Butane is then recovered as a product from this condenser.

Referring to Figure 2, it is seen how the cooling and heating functions of the present invention are applied to the processes that have depropaniser distillation columns. The numbers of similar lines and equipment that appear in Figure 1 have been kept the same.

The reactor effluent, after being used as a coolant, enters the first vapours liquid separator 117 through line 113. A liquid bottoms stream containing isobutane and alkylate is removed through line 136 and sent to further processing as shown in Figure 1. The vapours from the vapour liquid separator are transmitted through line 118 to compressor 119. The compressed vapours leave the compressor through line 120. The compressed vapours then go to provide heating in exchangers through lines 121 and 122. Specifically referring to line 121 it is seen how the present invention is applied to the depropaniser column. The compressed vapours are transmitted through line 121 to a heat exchanger 154 where at least part of the vapours are condensed to provide heat to the exchanger. The compressed vapours, after providing heat, are transmitted through line 126. This is combined with vapours that have exchanged heat in other exchangers returning in line 124, and transmitted to condenser 128. The condensed vapour stream leaves the condenser through line 130. A portion is taken through line 168 as feed to the depropaniser distillation column 152. Heat is applied to this column as described previously through heat exchanger 154. Propane is distilled and exits the top of the column as a vapour in line 156. This vapour is then condensed in condenser 158 by used of the reactor effluent in lines 112 and 114. The condensed propane is then split into line 160 as reflux to the column, and line 162 as propane product. The isobutane-rich bottoms leaves the depropaniser column through line 163, is let down in pressure through valve 164 and then transmitted to the second vapour liquid separator 132. The condensed vapours from condenser 128 that are not sent to the depropaniser column are also let down in pressure through valve 131 and transmitted to the second vapour liquid separator 132. The vapours are removed from this separator through line 133 and recycled back to the compressor as disclosed in Figure 1. The liquid bottoms stream 134, containing mostly isobutane, can be recycled back to the reactor as also shown in Figure 1.

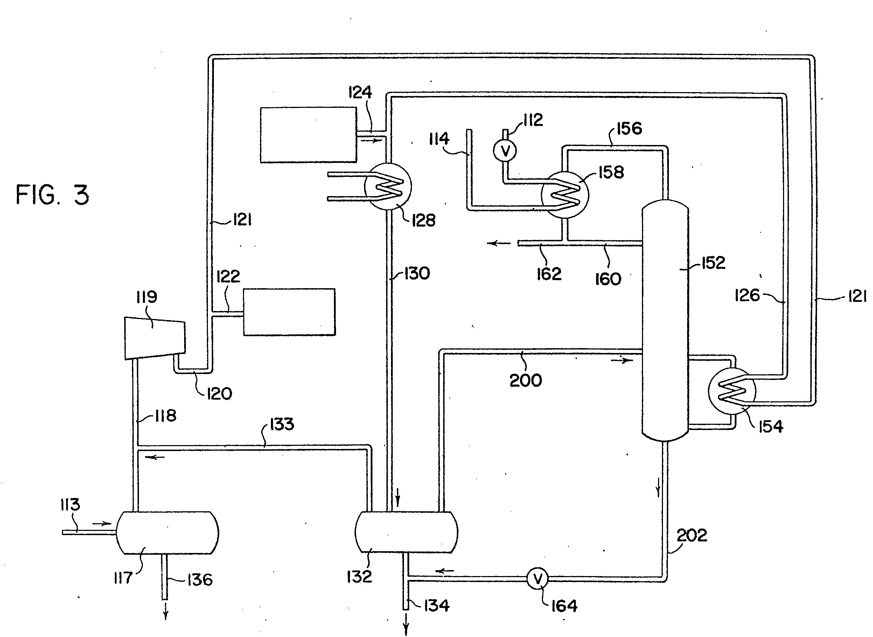

Figure 3 represents an alternative and preferred embodiment of the system shown in Figure 2.

The primary difference between Figures 2 and 3 is that the compressed vapours, after supplying heat to the depropaniser, are not totally condensed in condenser 128.

The partially condensed vapour stream leaves the condenser 128 through line 130 and is sent to the vapour-liquid separator 132. The uncondensed vapours are then transmitted through line 200 as feed to the depropaniser. Thus the feed to the depropaniser which is a liquid stream in the process of Figure 2 is now a vapour stream.

The isobutane-rich bottom stream leaves the depropaniser column through line 202 and is recycled back to the reactor. This stream can be combined with the isobutane in line 134 from the vapour-liquid separator.

The present invention has many advantages over the art. By combining the heating and cooling functions large energy savings may be acquired.

Cooling the overhead of the distillation columns by using the reactor effluent increases the amount of isobutane that is vapourised in the reactor effluent and need not be distilled. This reduces the size of the distillation columns and the amount of heat necessary to perform distillation. It also allows the distillation columns to be operated at a much lower pressure than presently exists in the art. By increasing the vapour flow and reducing the pressure in the distillation columns the compressed vapours can be used to supply the heat necessary for the distillation columns. Thus a significant saving is made in the cost of services because the water used to condense the overhead distillation column is no longer necessary, and the external heat supplied to the columns is greatly reduced or eliminated.

In a preferred embodiment of the process according to this invention, the reactor effluent, after acid separation is sent to the overhead condensers on the distillation columns of the process to provide cooling. The compressed vapours from the first liquid separator are sent to the bottom heat exchangers to the distillation columns to provide heat. This concept is preferred because lower temperatures can be achieved in the columns using the reactor effluent than by using cooling water. With lower temperature and pressures in the columns the separation process is greatly enhanced and the amount of heat input to achieve this separation is reduced.

Typically, at least two distillation columns are present, a deisobutaniser for separating isobutane from the alkylate product, and a depropaniser to recover the lighter propane from the isobutane. In some cases a third column for separating butane from alkylate is present. The use of the present invention on this tower is also contemplated. However, butane may also be removed as a sidestream draw from the deisobutaniser column as shown in the drawings.

After exchanging heat, the compressed vapour stream can be further condensed by a small cooler to remove the heat that cannot be economically recovered through heat exchange. After the stream is condensed, it can then be let down in pressure and sent to a vapour liquid separator. This separator removes as a vapour any uncondensed and lighter boiling components and recycles them back to the vapour compressor. The isobutane-rich liquid remaining in the separator can then be recycled back to the reactor.

The discharge pressure of the vapour compressor may be that generally used in known processes. However, in the process according to the invention a compressed vapour stream pressure of from 3.5 to 14 megabarye gauge is preferred, more preferably about 10.5 megabarye gauge. This pressure will vary according to the amount of heat transfer desired at a given distillation column. The higher the pressure of the distillation column the higher the discharge pressure of the vapour compressor. Thus it is advantageous to run the distillation columns at lower pressures than were achieved in the prior art.

The reactor illustrated in the Figures is a conventional liquid phase alkylation reactor. The reactor may however, also be a two phase cascade reactor in which the reaction zone is refrigerated directly by evaporation of low boiling hydrocarbons. In this type of reactor, the acid separation and vapour-liquid separation take place within the reactor itself. The vapours are removed from the reactor, compressed and then used to supply heat to the distillation columns. The liquid from the reactor, removed as a separate stream, can be used to condense the overhead of the distillation columns.

The deisobutaniser column may be operated at pressures between .07 and 10.5 megabarye gauge but it is preferred to operate at a pressure between 2.4 and 3.5 megabarye gauge. The depropaniser distillation column may be operated at a pressure between 3.5 and 10.5 megabarye gauge with a preferred pressure between 5.3 and 8.8 megabarye gauge.

This invention may be used in any catalytic alkylation process involving an acid catalyst. The acid catalysts that may be used are known in the art, in particular sulphuric acid and hydrofluoric acid. The use of a sulphuric acid catalyst is preferred in the process of the present invention.

The reaction conditions and parameters are not significantly changed by this invention. The reactor is normally operated between 0.07 and 14 megabarye gauge, and a temperature between −10 to 50°C.

The olefin feed to the alkylation process is also known in the art and is not changed by the process of the present invention. The composition of the olefin feed normally depends on the specific application, but may comprise propylene, butylenes or amylenes. The olefin feed may also contain various inert alkanes, such as propane and butane. The olefin is mixed with isobutane either before going to the reactor or in the reactor. Normally, the higher the ratio of isobutane to olefin in the feed stock the greater the yield of alkylate. This external ratio is usually about 5:1 but can be 15:1 or higher. The present invention takes advantage of this ratio by recovering isobutane for recycling in a more efficient and less costly manner than the prior art. By using this invention in existing units this ratio can be increased, thereby improving the octane of the product without being limited by the size of the deisobutaniser. Further, the use of a higher ratio also reduces acid consumption during the reaction.

As shown in the drawings, the reactor effluent used to provide cooling may be passed through a pressure-reducing valve prior to its entry into the various condensers. The pressure is reduced sufficiently to permit vapourisation and to effect greater cooling in the condenser. The pressures may be reduced to a pressure of 0.070 to 3.5 megabarye absolute. It is preferred to reduce this pressure to from .21 megabarye gauge to .35 megabarye gauge.

Example 1 and Comparative Example A

A computer simulation was made of an alkylation process as depicted in the *Hydrocarbon Processing* reference of September, 1974, page 206. Sulfuric acid was used as the acid catalyst. The reactant feed to both examples was 310,850 litres per stream day (LPSD), being a combination of isobutane, butylene and inert alkanes.

For comparison, the amount of product and its octane number were held constant for both examples. Due to the larger amounts of inert alkanes that are recycled in the effluent refrigeration stream of the present invention, the isobutane/olefin ratio in the reactor increased when compared to the art. The other operating conditions of the reactor were the same for both examples.

Comparative Example A shows the prior art. The reactor effluent after acid separation was used to cool the reactor. After cooling, the effluent stream was sent to the first vapour-liquid separator. The liquid from this separator constituted the stream that was treated and sent as feed to the deisobutaniser column. Cooling water was used as the cooling medium in the overhead condenser of this column. The vapours from the vapour liquid separator were compressed and condensed and used as feed to the depropaniser. Cooling water was used as the cooling medium in the overhead condenser of the depropaniser. Steam was used as an external heating source to both columns.

In Example 1, according to the present invention, the reactor effluent was sent in a parallel manner to the overhead condensers of the deisobutaniser and depropaniser columns. After cooling, the reactor effluent was collected in the first vapour liquid separator. The compressed vapours from the vapour liquid separator were used to supply the total heat to the depropaniser column. The vapours also supplied partial heat to the deisobutaniser column by using a side reboiler. The compressor discharge pressure using the present invention was 10.0 megabarye absolute. The reboiler temperatures for the depropaniser and the deisobutaniser columns were 53°C and 90.5°C respectively.

The results of these two experiments are shown in the following table.

TABLE 1
Comparison of Art Alkylation with Invention

| | Prior Art | Invention |
|---|---|---|
| Feed LPSD | 310,850 | 310,850 |
| $IC_4$/olefin ratio | 13.9 | 16.7 |
| *Deisobutaniser* | | |
| Feed LPSD | 1,621,650 | 996,300 |
| Column Diam. cm. | 230 | 140 |
| Top pressure megabarye abs. | 9.7 | 2.4 |
| External heat* | 8.14 | 0.63 |
| Heat from vapours* | — | 1.64 |
| Total heat* | 8.19 | 2.27 |
| *Depropaniser* | | |
| Feed LPSD | 481,135 | 717,565 |
| Top pressure megabarye abs. | 17.5 | 7 |
| External Heat | 1.13 | —0— |
| Heat from vapours | —0— | 1.16 |
| *Total External Heat* * | 9.45 | 3.43 |

*Cal × $10^6$/hour.

As shown in the table, far more of the isobutane contained in the reactor effluent is vapourised than is found in the prior art, allowing for a more efficient recovery. The feed to the deisobutaniser column decreased from 1,621,650 to 996,300 litres per stream day. This reduction in feed allowed for a much smaller column. The cooling function of the invention allowed the tower to be operated at a much lower pressure (2.4 megabarye absolute) and resulted in a 66% reduction in the total heat necessary to this column. The Table also shows that the heat from the compressed vapours can now supply a large amount of the heat necessary to reboil this column.

Although the feed to the depropaniser increased due to the increased amount of vapours produced, use of the cooling function of the invention allowed the tower pressure to be reduced from 17.5 to 7 megabarye absolute. The substantial reduction in pressure allowed the use of the compressed vapours to supply heat to this column. As the Table shows, all of the heat required by the depropaniser column can be supplied from the compressed vapours.

Of primary importance is the fact that the total heat to the process from an external source was reduced by 66%.

### Claims

1. A process for the catalytic alkylation of isobutane with an olefin in which
(a) the olefin is contacted with a molar excess of isobutane and inert alkanes in the presence of an acid catalyst in a reactor to form a reactor effluent containing alkylate, isobutane, acid catalyst and inert alkanes;
(b) the acid catalyst is separated from the reactor effluent to form a hydrocarbon mixture;
(c) a liquid vapour separation is effected on a hydrocarbon mixture to obtain a liquid bottoms stream containing isobutane and alkylate and an overhead vapour stream containing isobutane and inert alkanes and
(d) the liquid bottoms stream is heated in a distillation zone to form a liquid bottoms product containing alkylate and a vapour distillation effluent containing isobutane separated from the alkylate;
(e) a portion of the hydrocarbon mixture formed in step (b) is passed as a cooling effluent in indirect heat exchange with the vapour distillation effluent obtained in step (d) to condense isobutane and to vapourise at least a portion of the cooling effluent; and
(f) the cooling effluent is admixed with the hydrocarbon mixture formed in step (b) so that the cooling effluent is subjected to liquid vapour separation together with the hydrocarbon mixture in step (c); characterised in that the overhead vapour stream produced in step (c) is compressed in a compressor to obtain a compressed vapour stream and at least a portion of the compressed vapour stream is passed in indirect heat exchange relation with the liquid in the distillation zone of (d) to condense at least a portion of the compressed vapour stream and thereby supply heat to the distillation zone.

2. A process as claimed in claim 1 characterised in that a portion of the cooling effluent is partially vapourized by reducing the pressure of the cooling effluent prior to the indirect heat exchange.

3. A process as claimed in claim 1 or claim 2 characterised in that residual acid and impurities are removed from the liquid bottoms stream of (d) prior to distilling the stream.

4. A process as claimed in any of claims 1 to 3 characterised in that a portion of the condensed compressed vapour stream is recycled to the reactor.

5. A process as claimed in any of claims 1 to 4 characterised in that a portion of the compressed vapour stream is distilled in an alkane distillation zone to remove inert alkanes therefrom as a vapour alkane fraction, and the vapour alkane fraction from the alkane distillation is condensed by passing the vapour alkane fraction in indirect heat exchange with at least a portion of the cooling effluent.

6. A process as claimed in claim 5 characterised in that the inert alkane is propane.

7. A process as claimed in any of claims 1 to 6 characterised in that the liquid bottoms stream of (d) additionally contains normal butane and the distillation of the liquid bottoms stream includes the removal of normal butane as a vapour sidestream and condensing the vapour sidestreams by passing it in indirect heat exchange with a second portion of the cooling effluent.

8. A process as claimed in any of claims 5 to 7 characterised in that at least a portion of the compressed vapour stream is passed in indirect heat exchange with a liquid from the alkane distillation zone to condense at least a portion of the compressed vapour stream, thereby supplying heat to the alkane distillation zone.

9. A process as claimed in any of claims 1 to 8 in which the compressed vapour stream after supplying heat to the distillation zone, is further condensed by cooling.

10. A process as claimed in claim 9 characterised in that the condensed compressed vapour stream is sent to a second vapour liquid separator wherein a vapour stream is removed overhead and recycled to the compressor, and at least part of a liquid stream removed from the second vapour liquid separator is sent to the reactor as recycled isobutane.

11. A process as claimed in any of claims 1 to 10 characterised in that the compressed vapour stream is at a pressure of 3.5 to 14 megabarye gauge.

12. A process for separating inert alkanes and isobutane from a gaseous alkylation reactor effluent containing alkylate, isobutane and inert alkanes in which:
(a) a liquid vapour separation is effected on a portion of the reactor effluent to obtain an overhead vapour stream containing isobutane and inert alkanes and a liquid bottoms stream containing isobutane and alkylate and
(b) the overhead vapour stream is compressed; which process is characterised in that
(c) at least a portion of the compressed overhead vapour stream is passed through a heat exchanger thereby at least partially condensing the compressed overhead vapour stream to form an exchanger effluent stream;
(d) a first portion of the exchanger effluent stream is passed to an alkane distillation zone in which the liquid is heated by passing it in indirect heat exchange with the heat exchanger of (c), thereby distilling and separating inert alkanes as a vapour fraction and isobutane as a liquid bottoms product;
(e) a portion of the reactor effluent is passed as a cooling effluent in indirect heat exchange with the vapour fraction of (d) to condense the inert alkane therein and to vapourise a portion of the reactor effluent; and
(f) the cooling effluent is mixed with the reactor effluent so that the cooling effluent is subjected to liquid vapour separation together with the reactor effluent.

13. A process as claimed in claim 12 characterised in that the exchanger effluent stream of (c) is passed in indirect heat exchange in a condenser to condense totally the exchanger effluent stream prior to passing a portion of the effluent stream to the alkane distillation zone.

14. A process as claimed in claim 12 characterised in that a portion of the cooling effluent of (e) is partially vapourised by reducing its pressure prior to the indirect heat exchange.

15. A process as claimed in claim 12 characterised in that the liquid bottoms product of (d) containing isobutane, and a second portion of the exchanger effluent stream of (c) containing isobutane and inert alkanes, are reduced in pressure and passed to a second vapour liquid separator, thereby forming a second overhead vapour stream containing isobutane and inert alkanes and a second liquid bottom stream containing isobutane.

16. A process as claimed in any of claims 12 to 15 characterised in that the inert alkane is propane.

17. A process as claimed in any of claims 1 to 16 characterised in that the olefin is propylene, butylene or an amylene.

18. A process as claimed in any of claims 1 to 17 characterised in that the acid catalyst is sulphuric acid.

19. A process as claimed in any of claims 12 to 18 characterised in that the portion of the exchanger effluent stream of (d) sent to the alkane distillation zone is vapour.

**Patentansprüche**

1. Verfahren zur katalytischen Alkylierung von Isobutan mit einem Olefin, wobei
(a) das Olefin mit einem molekularen Überschuß an Isobutan und inerten Alkanen in Anwesenheit eines Säure-Katalysators in einem Reaktor unter Bildung eines ein Alkylat, Isobutan, Säure-Katalysator und inerte Alkane enthaltenden Reaktorabgangsproduktes in Berührung gebracht wird;
(b) der Säure-Katalysator vom Reaktorabgangsprodukt unter Bildung eines Kohlenwasserstoff-Gemisches abgetrennt wird;

(c) eine Flüssigkeit-Dampf-Trennung mit dem Kohlenwasserstoff-Gemisch durchgeführt wird, wodurch ein Isobutan und Alkylat enthaltendes flüssiges Bodenabgangsprodukt und ein Isobutan und inerte Alkane enthaltendes dampfförmiges Kopfprodukt erhalten wird; und

(d) das flüssige Bodenabgangsprodukt in einer Destillationszone erhitzt wird, wodurch ein Alkylat enthaltendes flüssiges Bodenprodukt und ein von dem Alkylat abgetrenntes, Isobutan enthaltendes dampfförmige Destillat erhalten wird;

(e) ein Anteil des in der Stufe (b) gebildeten Kohlenwasserstoff-Gemisches als Kühlabgas in indirektem Wärmeaustausch mit dem in Stufe (d) gebildeten dampfförmigen Destillat gebracht wird, um Isobutan zu kondensieren und zumindest ein Teil des Kühlmittels zu verdampfen; und

(f) das Kühlmittel mit dem in der Stufe (b) gebildeten Kohlenwasserstoff-Gemisch gemischt wird, so daß das Kühlmittel der Flüssigkeit-Dampf-Trennung zusammen mit dem Kohlenwasserstoff-Gemisch in Stufe (c) unterworfen wird, dadurch gekennzeichnet, daß der in Stufe (c) gebildete, am Kopf abgenommene dampfförmige Produktenstrom in einem Kompressor komprimiert wird, wordurch ein komprimierter Dampfstrom erhalten wird, und mindestens ein Teil des komprimierten Dampfstromes in indirektem Wärmeaustausch mit der Flüssigkeit in der Destillationszone der Stufe (d) gebracht wird, um mindestens ein Teil des komprimierten Dampfstromes zu kondensieren und hierdurch der Destillationszone Wärme zuzuführen.

2. Verfahren gemäß Anspruch 1, dadurch gekennzeichnet, daß ein Teil des flüssigen Kühlmittels durch Verminderung des Drucks des Kühlmittels vor dem indirekten Wärmeaustausch teilweise verdampft.

3. Verfahren gemäß Anspruch 1 und 2, dadurch gekennzeichnet, daß Restsäure und Verunreinigungen von dem flüssigen Bodenprodukt der Stufe (d) vor der Destillation des Produktenstromes abgetrennt werden.

4. Verfahren gemäß irgendeinem der Ansprüche 1 bis 3, dadurch gekennzeichnet, daß ein Teil des kondensierten komprimierten Dampfstromes zu dem Reaktor wieder zurückgeführt wird.

5. Verfahren gemäß irgendeinem der Ansprüche 1 bis 4, dadurch gekennzeichnet, daß ein Teil des komprimierten Dampfstromes in einer Alkan-Destillationszone destilliert wird, um inerte Alkane hieraus als dampfförmige Alkan-Fraktion zu entfernen, und daß die dampfförmige Alkan-Fraktion der Alkan-Destillation kondensiert wird, indem die dampfförmige Alkan-Fraktion in indirektem Wärmeaustausch mit mindestens einem Teil des Kühlmittels gebracht wird.

6. Verfahren gemäß Anspruch 5, dadurch gekennzeichnet, daß das inerte Alkan Propan ist.

7. Verfahren gemäß irgendeinem der Ansprüche 1 bis 6, dadurch gekennzeichnet, daß das flüssige Bodenprodukt der Stufe (d) zusätzlich n-Butan enthält und die Destillation des flüssigen Bodenproduktes die gleichzeitige Entfernung des n-Butans als dampfförmiger Seitenstrom umfaßt und die dampfförmigen Seitenströme kondensiert werden, indem sie in indirekten Wärmeaustausch mit einem zweiten Teil des Kühlmittels gebracht werden.

8. Verfahren gemäß irgendeinem der Ansprüche 5 bis 7, dadurch gekennzeichnet, daß mindestens ein Teil des komprimierten Dampfstromes in indirekten Wärmeaustausch mit einer Flüssigkeit von der Alkan-Destillationszone gebracht wird, um mindestens einen Teil des komprimierten Dampfstromes zu kondensieren, wodurch Wärme der Alkan-Destillationszone zugeführt wird.

9. Verfahren gemäß irgendeinem der Ansprüche 1 bis 8, dadurch gekennzeichnet, daß der komprimierte Dampfstrom durch Kühlen weiter kondensiert wird, nachdem Wärme der Destillationszone zugeführt wurde.

10. Verfahren gemäß Anspruch 9, dadurch gekennzeichnet, daß der kondensierte komprimierte Dampfstrom einer zweiten Dampf-Flüssigkeit-Abtrennvorrichtung zugeführt wird, worin ein Dampfstrom über Kopf abgetrennt und dem Kompressor wieder zugeführt wird und mindestens ein Teil des von der zweiten Dampf-Flüssigkeit-Abtrennvorrichtung abgezogenen flüssigen Produktenstromes dem Reaktor als zurückgeführtes Isobutan zugeführt wird.

11. Verfahren gemäß irgendeinem der Ansprüche 1 bis 10, dadurch gekennzeichnet, daß der komprimierte Dampfstrom einen Druck von 3,5 bis 14 megabar hat.

12. Verfahren zur Abtrennung inerter Alkane und Isobutan aus dem Alkylat, Isobutan und inerte Alkane enthaltenden gasförmigen Abgas aus einem Alkylierungsreaktor, wobei

(a) eine Flüssigkeit-Dampf-Abtrennung mit einem Teil der Reaktorabgase durchgeführt wird, wodurch ein über Kopf abgehender, Isobutan und inerte Alkane enthaltender Dampfstrom und ein Isobutan und Alkylat enthaltendes flüssiges Bodenprodukt erhalten wird und

(b) der über Kopf abgehende Dampfstrom komprimiert wird,

dadurch gekennzeichnet, daß

(c) mindestens ein Teil des komprimierten, über Kopf abgehenden Dampfstromes durch einen Wärmeaustauscher geleitet wird, wodurch mindestens eine teilweise Kondensierung des komprimierten, über Kopf abgehenden Dampfstromes unter Bildung eines Wärmeaustauscherabgangsproduktes erhalten wird;

(d) ein erster Anteil des Wärmeaustauscherabgangsproduktes einer Alkan-Destillationszone zugeführt wird, in der das flüssige Produkt erhitzt wird, indem es in indirektem Wärmeaustausch mit dem Wärmeaustauscher der Stufe (c) gebracht wird, wodurch inertes Alkan als dampfförmige Fraktion und Isobutan als flüssiges Bodenprodukt destilliert und abgetrennt wird;

(e) ein Teil des Reaktorabgangsproduktes als Kühlungsmittel einem indirekten Wärmeaustausch mit der dampfförmigen Fraktion der Stufe (d) zugeführt wird, wodurch das hierin enthaltene inerte Alkan kondensiert und ein Teil des Reaktorabgangsproduktes verdampft wird; und

(f) das Kühlmittel mit dem Reaktorabgangsprodukt vermischt wird, so daß das Kühlmittel zusammen mit dem Reaktorabgangsprodukt der Flüssigkeit-Dampf-Auftrennung unterworfen wird.

13. Verfahren gemäß Anspruch 12, dadurch gekennzeichnet, daß das Wärmeaustauscherabgangsprodukt der Stufe (c) ein einem indirekten Wärmeaustausch in einem Kondensator unterworfen wird, um so die Gesamtmenge des Wärmeaustauscherabgangsproduktes zu kondensieren, bevor ein Teil des Abgangsdampfes der Alkan-Destillationszone zugeführt wird.

14. Verfahren gemäß Anspruch 12, dadurch gekennzeichnet, daß ein Teil des Kühlmittels der Stufe (e) teilweise verdampft wird, indem sein Druck vermindert wird, bevor es dem indirekten Wärmeaustausch unterworfen wird.

15. Verfahren gemäß Anspruch 12, dadurch gekennzeichnet, daß das Isobutan enthaltende, flüssige Bodenprodukt der Stufe (d) und ein zweiter Teil des Isobutan und inerte Alkane enthaltenden Wärmeaustauscherabgangsproduktes der Stufe (c) einer Druckverminderung unterworfen und einer zweiten Dampf-Flüssigkeit-Abtrennvorrichtung zugeführt wird, so daß hierdurch ein zweiter, dampfförmiges Isobutan und inerte Alkane enthaltender Dampfstrom und ein zweiter, Isobutan enthaltender flüssiger Bodenstrom erhalten wird.

16. Verfahren gemäß irgendeinem der Ansprüche 12 bis 15, dadurch gekennzeichnet, daß das inerte Alkan Propan ist.

17. Verfahren gemäß irgendeinem der Ansprüche 1 bis 16, dadurch gekennzeichnet, daß das Olefin Propylen, Butylen oder Amylen ist.

18. Verfahren gemäß irgendeinem der Ansprüche 1 bis 17, dadurch gekennzeichnet, daß der Säure-Katalysator Schwefelsäure ist.

19. Verfahren gemäß irgendeinem der Ansprüche 12 bis 18, dadurch gekennzeichnet, daß der Anteil des Wärmeaustauscherabgangsproduktes der Stufe (d), der der Alkan-Destillationszone zugeführt wird, Dampf ist.

**Revendications**

1. Procédé d'alcoylation catalytique de l'isobutane par une oléfine dans lequel:

(a) on met en contact l'oléfine avec une excès molaire d'isobutane et d'alcanes inertes en présence d'un catalyseur acide dans un réacteur pour former un effluent de réacteur contenant le produit d'alcoylation, l'isobutane, le catalyseur acide et des alcanes inertes;

(b) on sépare le catalyseur acide à partir de l'effluent du réacteur pour former un mélange d'hydrocarbures;

(c) on effectue une séparation liquide vapeur sur le mélange d'hydrocarbures de manière à obtenir un courant liquide des queues de distillation contenant l'isobutane et le produit d'alcoylation et un courant de vapeur de tête de distillation contenant l'isobutane et des alcanes inertes et

(d) on chauffe le courant liquide des queues de distillation dans une zone de distillation pour former un produit résiduel liquide contenant le produit d'alcoylation et un effluent de distillation sous forme de vapeur contenant de l'isobutane séparé à partir du produit d'alcoylation;

(e) on soumet une partie du mélange hydrocarboné formé dans l'étape (b) comme effluent de refroidissement à un échange de chaleur indirect avec l'effluent de distillation sous forme vapeur obtenue dans l'étape (d) pour condenser l'isobutane et vaporiser au moins une partie de l'effluent de refroidissement; et

(f) on mélange l'effluent de refroidissement avec le mélange d'hydrocarbures formé dans l'étape (b) de sorte que l'effluent de refroidissement soit soumis à une séparation liquide vapeur avec le mélange d'hydrocarbures dans l'étape (c), caractérisé par le fait que le courant de tête de distillation sous forme vapeur produit dans l'étape (c) est comprimé dans un compresseur pour obtenir un courant de vapeur comprimée et au moins une partie du courant de vapeur comprimée est soumise à un échange de chaleur indirect avec le liquide dans la zone de distillation de (d) pour condenser au moins une partie du courant de vapeur comprimée et fournir ainsi de la chaleur à la zone de distillation.

2. Procédé selon la revendication 1, caractérisé par le fait qu'une partie de l'effluent de refroidissement est partiellement vaporisée en réduisant la pression de l'effluent de refroidissement antérieurement à l'échange de chaleur indirect.

3. Procédé selon l'une des revendications 1 ou 2, caractérisé par le fait qu'on élimine de l'acide résiduel et des impuretés du courant liquide des queues de distillation de (d) avant la distillation de ce courant.

4. Procédé selon l'une des revendications 1 à 3, caractérisé par le fait qu'une partie du courant de vapeur comprimée condensée est recyclée dans le réacteur.

5. Procédé selon l'une des revendications 1 à 4, caractérisé par le fait qu'une partie du courant de vapeur comprimée est distillée dans une zone de distillation d'alcane pour en séparer les alcanes inertes sous forme d'une fraction vapeur d'alcanes et que cette fraction vapeur d'alcanes provenant de la distillation des alcanes des condensée en la soumettant à un échange de chaleur indirect avec au moins une partie de l'effluent de refroidissement.

6. Procédé selon l'une des revendications 1 à 5, caractérisé par le fait que l'alcane inerte est du propane.

7. Procédé selon l'une des revendications 1 à 6, caractérisé par le fait que le courant liquide des queues de distillation de (d) contient, en outre, du butane normal et la distillation du courant liquide des queues de distillation comprend l'élimination du butane normal comme courant latéral de vapeur et la condensation des courants latéraux de vapeur en les soumettant à un échange indirect de chaleur avec une seconde partie de l'effluent de refroidissement.

8. Procédé selon l'une des revendications 5 à 7, caractérisé par le fait qu'une partie au moins du courant de vapeur comprimée est soumise à un échange de chaleur indirect avec un liquide provenant de la zone de distillation d'alcane pour condenser au moins une partie du courant de vapeur comprimée, de la chaleur étant ainsi fournie à la zone de distillation d'alcane.

9. Procédé selon l'une des revendications 1 à 8, caractérisé par le fait que le courant de vapeur comprimée est en outre condensé par refroidissement après avoir fourni de la chaleur à la zone de distillation.

10. Procédé selon la revendication 9, caractérisé par le fait que le courant de vapeur comprimée condensée est envoyé dans un second séparateur liquide vapeur, un courant de vapeur étant séparé en tête de colonne et recyclé dans le compresseur, et qu'au moins une partie d'un courant liquide éliminé du second séparateur liquide vapeur est envoyée dans le réacteur comme isobutane recyclé.

11. Procédé selon l'une des revendications 1 à 10, caractérisé par le fait que le courant de vapeur comprimée est à une pression manométrique comprise entre 3,5 et 14 mégabaryes.

12. Procédé de séparation d'alcanes inertes et d'isobutane à partir d'un effluent gazeux de réacteur d'alcoylation contenant le produit d'alcoylation, l'isobutane et des alcanes inertes, dans lequel:

(a) on effectue une séparation liquide vapeur sur une partie de l'effluent du réacteur pour obtenir un courant de tête de distillation sous forme vapeur contenant l'isobutane et des alcanes inertes et un courant liquide de queues de distillation contenant l'isobutane et le produit d'alcoylation et

(b) on comprime le courant de vapeur de tête, ce procédé étant caractérisé par le fait que

(c) au moins une partie du courant de vapeur comprimée de tête passe par un échangeur de chaleur et est ainsi au moins partiellement condensée pour former un courant d'effluent de l'échangeur;

(d) on fait passer une première partie du courant de l'effluent de l'échangeur dans une zone de distillation d'alcane dans laquelle on chauffe le liquide en le soumettant à un échange de chaleur indirect dans l'échangeur de chaleur de (c) l'alcane inerte étant ainsi distillé et séparé sous forme d'une fraction vapeur et de l'isobutane étant séparé sous forme de produit des queues de distillation liquides.

(e) on fait passer une partie de l'effluent du réacteur comme effluent de refroidissement pour le soumettre à un échange indirect de chaleur avec le fraction vapeur de (d) pour condenser l'alcane inerte qui y est contenu et vaporiser une partie de l'effluent du réacteur; et

(f) on mélange l'effluent de refroidissement avec l'effluent du réacteur de sorte que l'effluent de refroidissement soit soumis à une séparation liquide vapeur avec l'effluent du réacteur.

13. Procédé selon la revendication 12, caractérisé par le fait que le courant de l'effluent de l'échangeur de (c) est soumis à un échange indirect de chaleur dans un condenseur pour condenser totalement le courant de l'effluent de l'échangeur avant passage d'une partie du courant de l'effluent dans la zone de distillation d'alcane.

14. Procédé selon la revendication 12, caractérisé par le fait qu'on vaporise partiellement une partie de l'effluent de refroidissement de (e) par diminution de sa pression avant l'échange indirect de chaleur.

15. Procédé selon la revendication 12 caractérisé par le fait que l'on soumet à une réduction de pression le produit des queues de distillation liquides de (d) contenant l'isobutane et une seconde partie du courant de l'effluent de l'échangeur de (c) contenant l'isobutane et des alcanes inertes et qu'on les amène dans un second séparateur liquide vapeur en formant ainsi un second courant de vapeur contenant l'isobutane et des alcanes inertes et un second courant liquide de queues de distillation contenant l'isobutane.

16. Procédé selon l'une des revendications 12 à 15, caractérisé par le fait que l'alcane inerte est du propane.

17. Procédé selon l'une des revendications 1 à 16, caractérisé par le fait que l'oléfine est du propylène, du butylène ou un amylène.

18. Procédé selon l'une des revendications 1 à 17 caractérisé par le fait que le catalyseur acide est l'acide sulfurique.

19. Procédé selon l'une des revendications 12 à 18, caractérisé par le fait que le partie du courant de l'effluent de l'échangeur de (d), envoyée dans la zone de distillation d'alcane, est de la vapeur.

FIG. 1

0 002 313

FIG. 2

FIG. 3

0002313